# EUROPEAN PATENT SPECIFICATION

(11) **EP 1 051 179 B1**
(45) Date of publication and mention of the grant of the patent: **14.09.2005**
(21) Application number: 98956525.4
(22) Date of filing: 04.11.1998
(51) Int. Cl.: A61K 31/57, A61K 31/565, A61K 31/55, A61K 31/495, A61K 31/40

(54) **ANTI-ESTROGEN PLUS PROGESTIN CONTAINING ORAL CONTRACEPTIVES**
ANTI-ÖSTROGEN- UND PROGESTIN-ENTHALTENDE ORALE KONTRAZEPTIVA
CONTRACEPTIFS ORAUX A BASE D'ANTI-OESTROGENES ASSOCIES A UN PROGESTATIF

(30) Priority: 06.11.1997 US 965083
(43) Date of publication of application: 15.11.2000
(73) Proprietor: Wyeth, Madison, New Jersey 07940-0874 (US)
(72) Inventor: GAST, Michael, Jay, Phoenixville, PA 19460 (US); MILLER, Christopher, Paul, Wayne, PA 19087 (US)
(74) Representative: Mannion, Sally Kim
(86) International application number: PCT/US1998/023427
(87) International publication number: WO 1999/024027

(56) References cited:
- EP-A- 0 791 356
- EP-A- 0 792 641
- EP-A- 0 802 183
- WO-A-96/19997
- GB-A- 1 326 528
- US-A- 5 747 480

## Description

### BACKGROUND

This invention relates to oral contraceptive regimens containing a non-uterotrophic antiestrogen (i.e., a tissue selective estrogen) and a progestin.

The vast majority of oral contraceptives consist of a combination of a progestin and estrogen that are administered concurrently for 21 days followed either by a 7 day pill free interval or by the administration of a placebo for 7 days in each 28 day cycle. The most important aspects of a successful oral contraceptive product are effective contraception, good cycle control (absence of spotting and breakthrough bleeding and occurrence of withdrawal bleeding), and minimal side effects. Combination oral contraceptives have traditionally acted by suppression of gonadotropins. In addition, it appears that the progestin component is primarily responsible for contraceptive efficacy through inhibition of ovulation, and other peripheral effects which include changes in the cervical mucus (which increase the difficulty of sperm entry into the uterus) and the endometrium (which reduce the likelihood of implantation). The estrogenic component intensifies the anovulatory effect of the progestin, and is also important for maintaining cycle control.

Several examples of progestin only contraceptives are known. For example products containing norethindrone (350 µg) or levonorgestrel (75 µg) are available, but raise several issues which limit their ultimate acceptability. The first is that currently available oral progestin only contraceptives are administered at doses that fail to completely inhibit ovulation thus pregnancy rates are marginally higher than currently available combined oral contraceptive preparations. Nonetheless, the pregnancy rates (generally less than 3 per 100 women per year) are excellent and are based primarily on cervical mucus changes and modest changes in endometrium. The second difficulty with these preparations is the extraordinarily high rate of abnormal or unexpected vaginal bleeding in women who utilize them. The absence of predictable vaginal bleeding which results from irregular development and shedding of the uterine lining (endometrium) is a phenomenon that is common to injectable, implantable and oral progestin only contraceptives. This side-effect is reported by up to 80% of women using any of these forms of progestin only contraception.

GB Patent Specification 1326528 discloses estrogen antagonizing agents (preferably cis-clomiphene) in combination with a progestin for use as a contraceptive. The estrogen antagonists disclosed in GB 1326528 are uterotrophic (see Kumar, A. India. J. Biosc. 20(5): 665 (1995)) whereas the anti-estrogens of this invention are not.

### DESCRIPTION OF THE INVENTION

This invention provides a contraceptive regimen for females of child-bearing age which comprises administering a combination of a non-uterotrophic anti-estrogen and a progestin continuously during the 28-day menstrual cycle. Non-uterotrophic antiestrogens are defined as those which typically will not produce clinically significant endometrial proliferation. More particularly, this invention provides a method providing contraception to a female of child bearing age which comprises administering a contraceptive effective amount of a combination of a non-uterotrophic anti-estrogen of formulas I or II having the structures 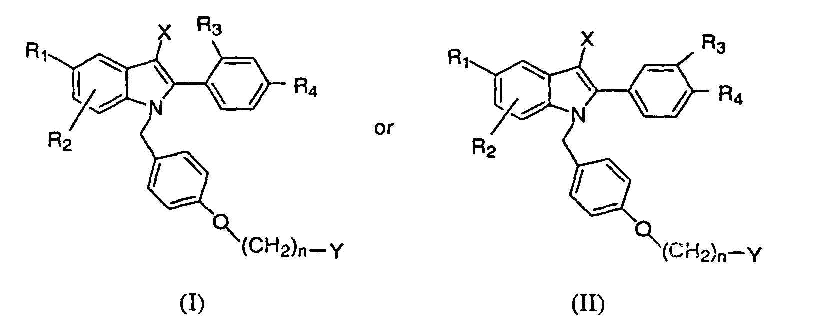 wherein:
- R₁: is H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen or mono- or poly-fluoroalkoxy of 1-12 carbon atoms;
- R₂: is H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen, mono- or poly-fluoroalkoxy of 1-12 carbon atoms, cyano, alkyl of 1-6 carbon atoms, or trifluoromethyl, with the proviso that, when R₁ is H, R₂ is not OH;
- R₃ and R₄: are each, independently, H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen, mono- or poly-fluoroalkoxy of 1-12 carbon atoms, or cyano;
- X: is H, alkyl of 1-6 carbon atoms, cyano, nitro, trifluoromethyl, or halogen;
- n: is 2 or 3;
- Y: is a saturated, partially saturated or unsaturated 5-7 membered heterocycle containing a nitrogen, which may optionally contain a second heteroatom selected from the group consisting of -O-, -NH- , alkylamine of 1-6 carbon atoms, -N< , and S(O)ₘ;
- m: is 0-2;
or a pharmaceutically acceptable salt thereof,
and a progestin for 28 consecutive days per 28-day menstrual cycle.

When any of R₁ to R₄ are aralkoxycarbonyl or aralkoxy, the aryl group is preferably benzyl. When any of R₁ to R₄ are cycloalkyloxy, the cycloalkyl group is preferably cyclopentyl. When any of R₁ to R₄ are alkoxy, the alkyl group is preferably methyl, ethyl, propyl or butyl, straight or branched chained. n is preferably 2.

Preferred compounds are those in which
- R₁: is selected from H, OH, alkoxycarbonyl of 2-12 carbon atoms, alkoxy of 1-12 carbon atoms, benzyloxy, cycloalkyloxy of 3-12 carbon atoms or halogen;
- R₂, R₃ and R₄: are independently selected from H, OH or alkoxycarbonyl of 2-12 carbon atoms, alkoxy of 1-12 carbon atoms, benzyloxy, cycloalkyloxy of 3-12 carbon atoms, halogen, cyano, C₁-C₆ alkyl, or trihalomethyl, preferably trifluoromethyl, with the proviso that, when R₁ is H, R₂ is not OH;
- X: is selected from H, C₁-C₆ alkyl, cyano, nitro, trifluoromethyl, halogen;
and Y is

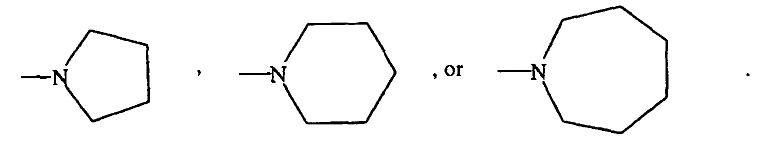

The preparation of the anti-estrogens of Formulas I and II is disclosed in EP-A-8 802 183.

Specifically preferred anti-estrogens of formulas I and II are shown in the Tables below.

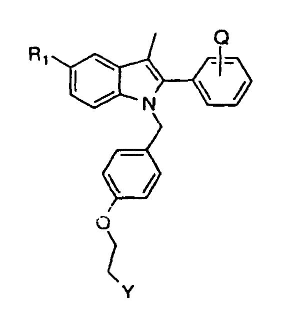

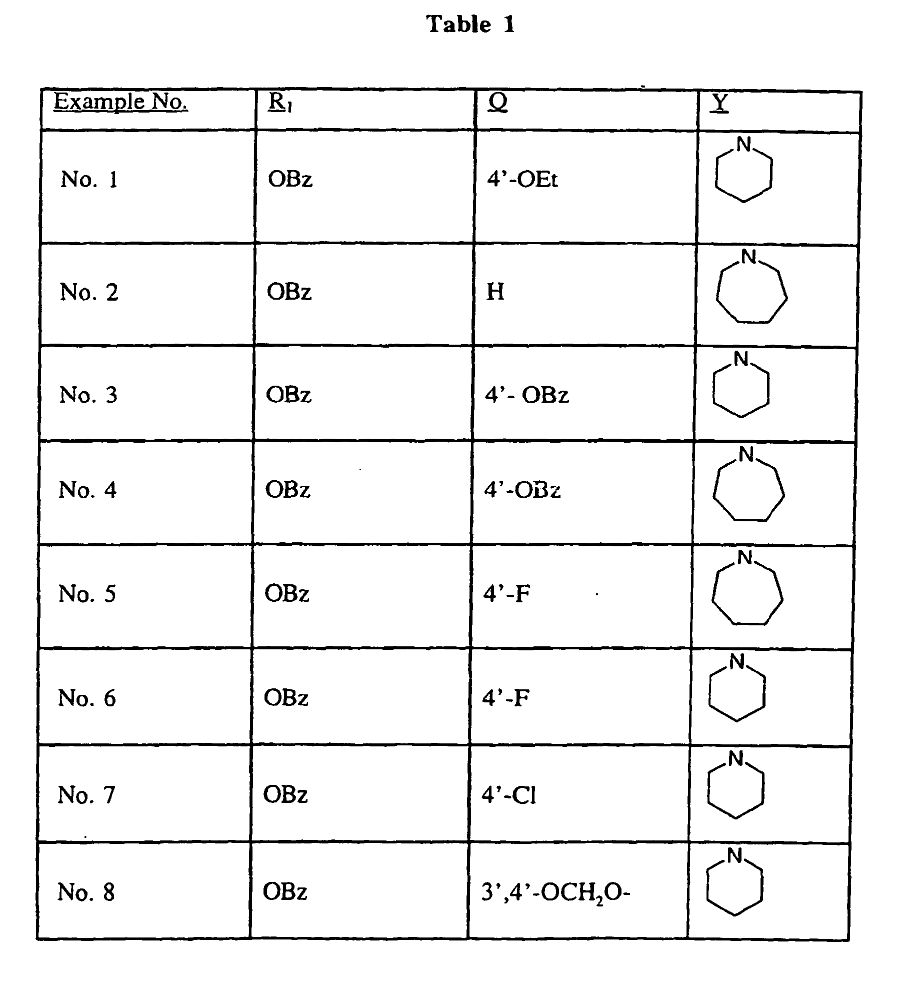

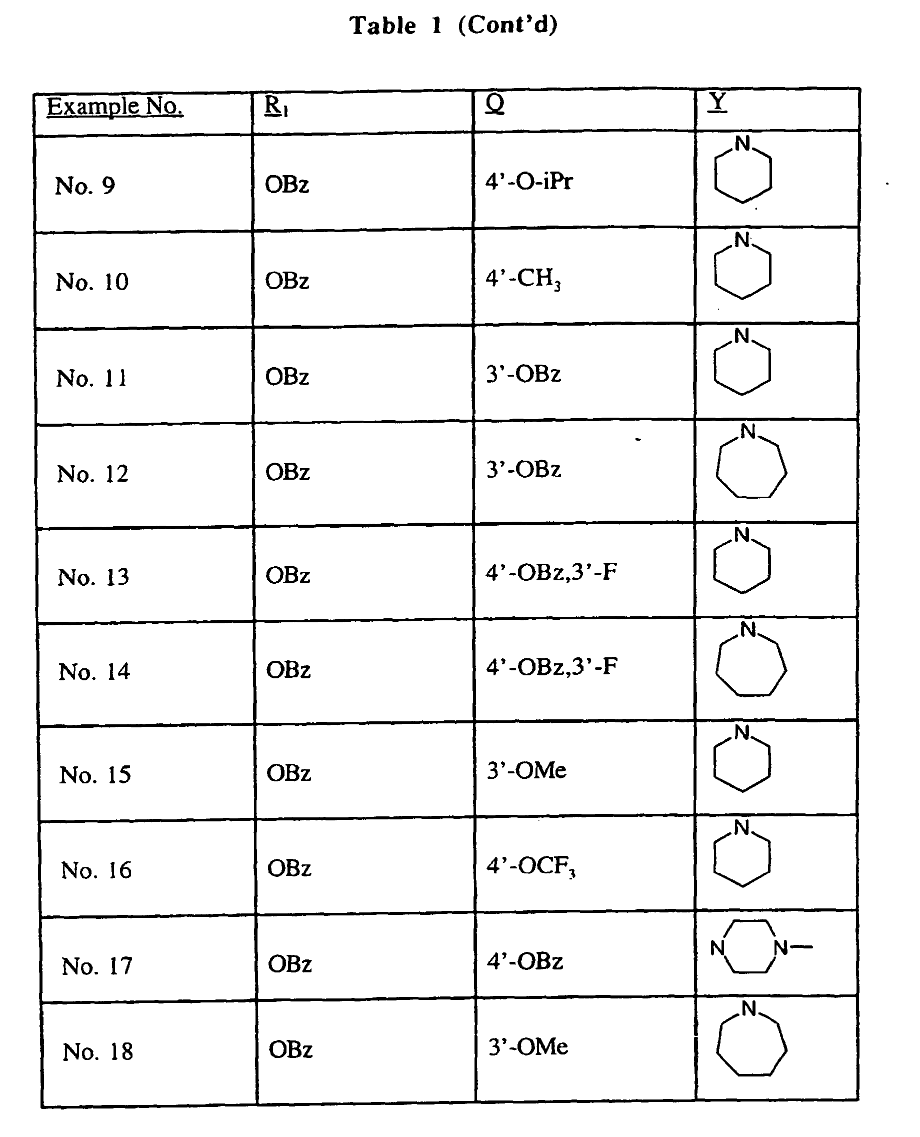

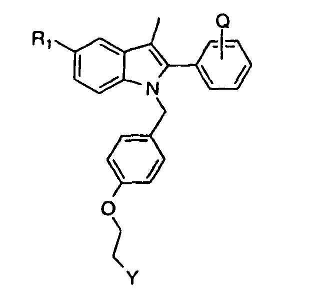

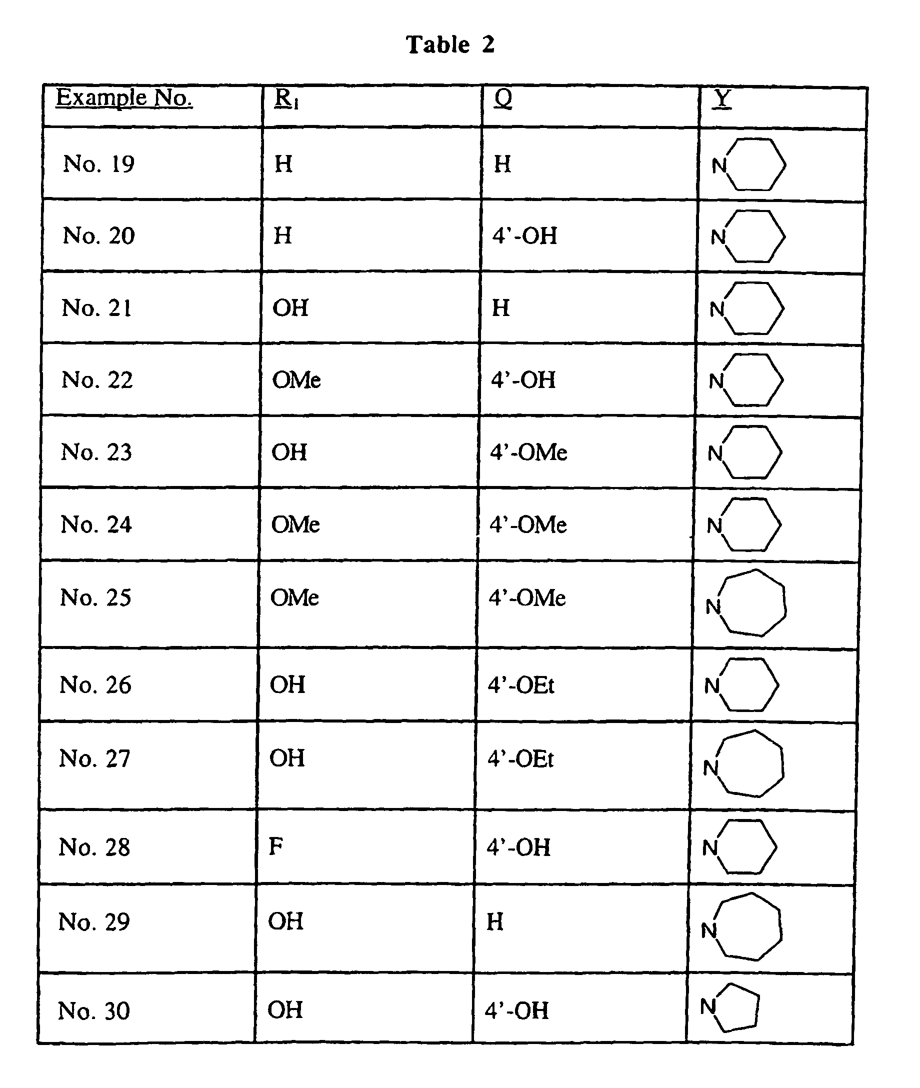

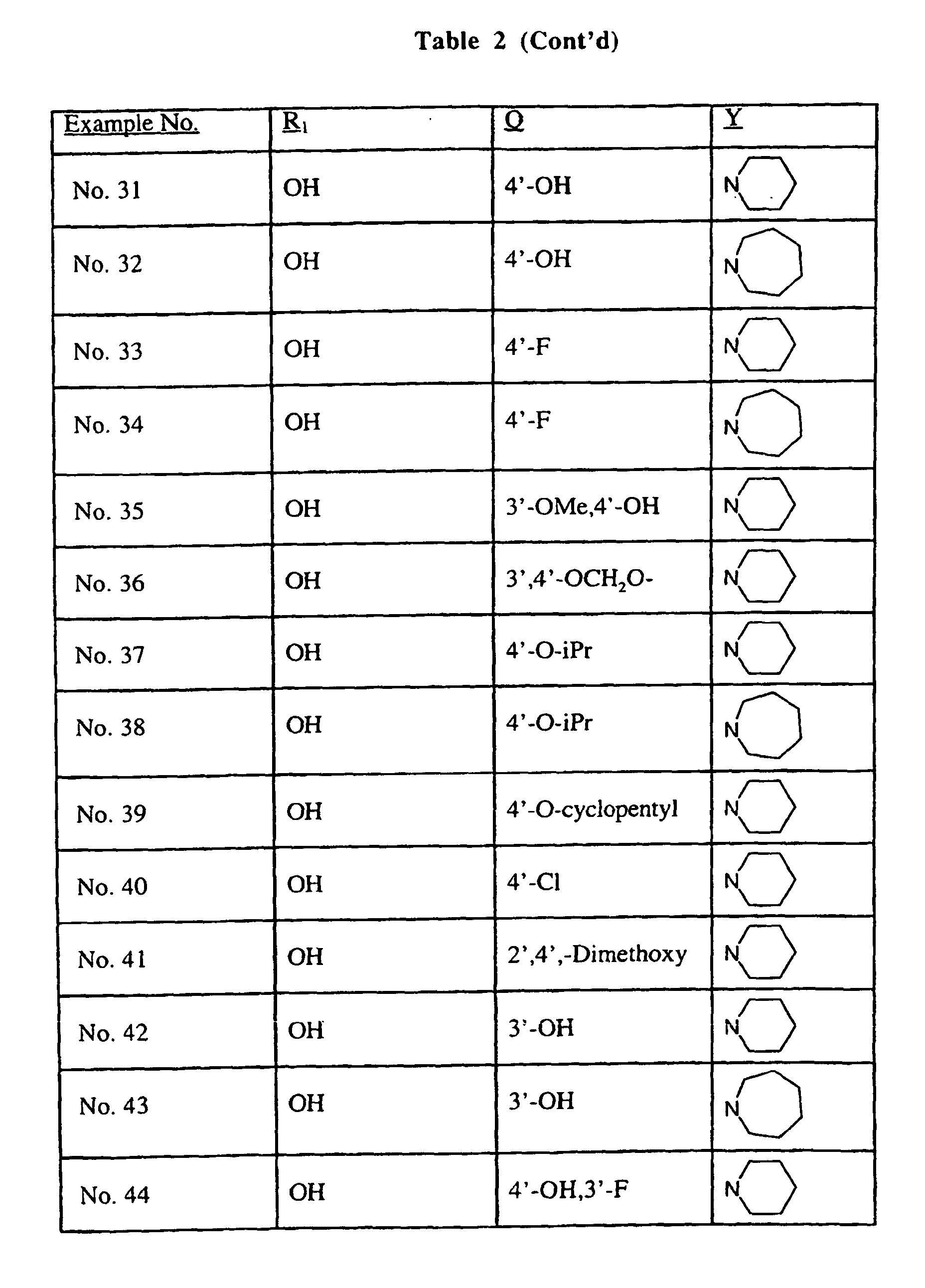

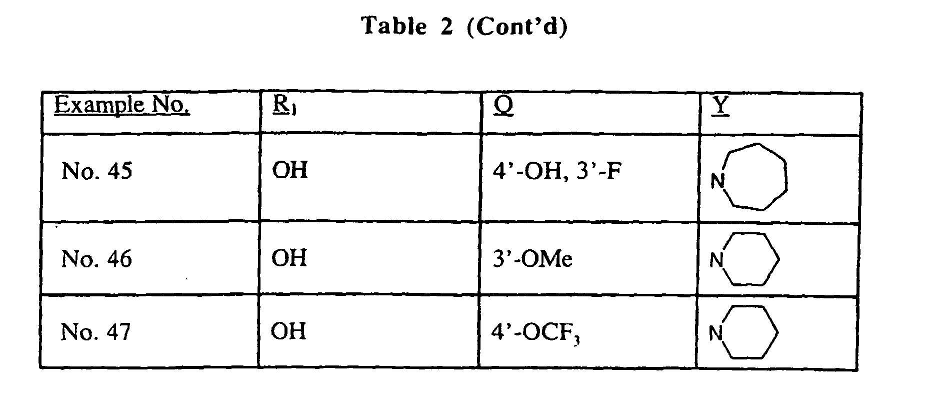

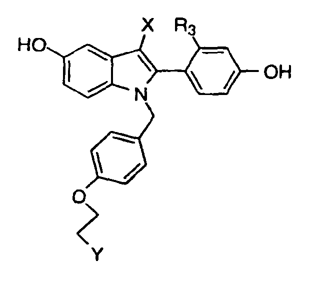

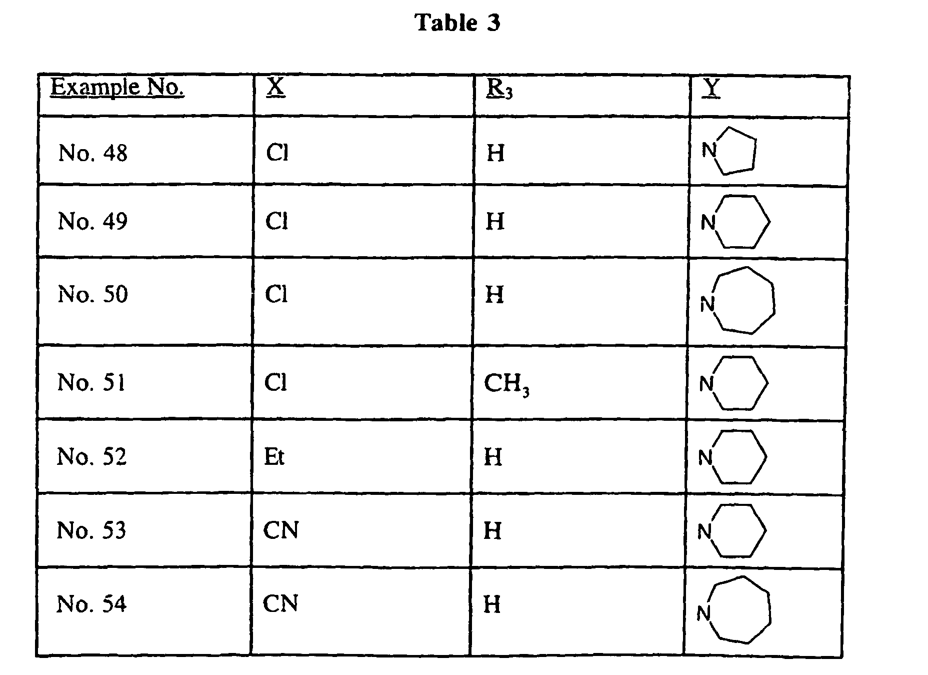

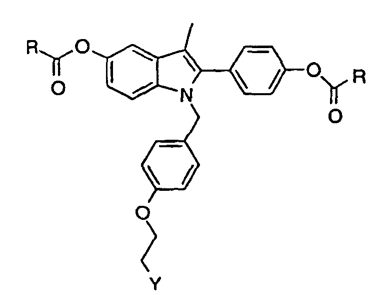

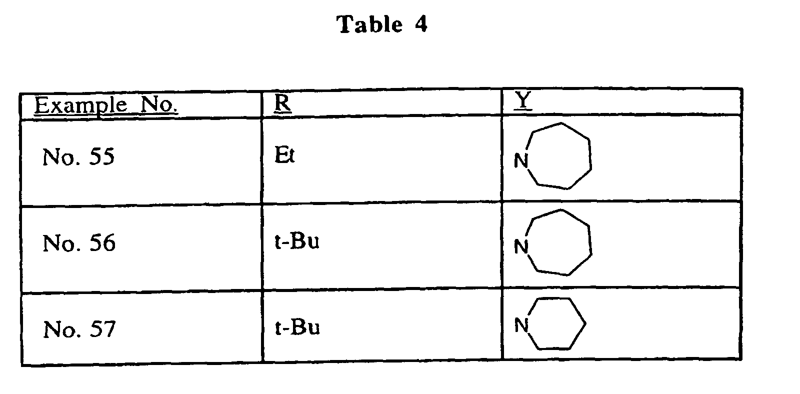

Particularly preferred anti-estrogens of Formulas I or II are those of Examples 31 and 32 in the tables above. It is preferred that the anti-estrogen of Formulas I or II is administered at a daily dosage equivalent to 0.1 - 150 mg of the compound of Example 32.

Preferred progestins include levonorgestrel, norgestrel, desogestrel, 3-ketodesogestrel, norethindrone, gestodene, norethisterone acetate, norgestimate, osaterone, cyproterone acetate, trimegestone, dienogest, and drospirenone. It is more preferred that the progestin is levonorgestrel. When levonorgestrel is used as the progestin, it is preferred that the daily dosage of levonorgestrel is 30-150 µg, with 50-110 µg being more preferred, and 75-100 µg being most preferred. The following table shows the preferred dosages of representative progestins of this invention.

| PREFERRED PROGESTIN DAILY DOSAGE RANGES | |
|---|---|
| Progestin | Dosage |
| Levonorgestrel | 30-150 µg |
| Norgestrel | 60-300 µg |
| Desogestrel | 45-225 µg |
| 3-Ketodesogestrel | 45-225 µg |
| Norethindrone | 100 µg - 1 mg |
| Norethisterone Acetate | 100 µg - 1 mg |
| Gestodene | 20-115 µg |
| Norgestimate | 75-500 µg |
| Osaterone | 100 µg - 2.5 mg |
| Trimegestone | 30-1500 µg |
| Dienogest | 500 µg - 3.75 mg |
| Drospirenone | 500 µg - 3.75 mg |
| Cyproterone Acetate | 450 µg - 2.5 mg |

This invention also covers the administration of a combination of other non-uterotrophic anti-estrogens (and preferred daily dosages), such as raloxifene (1-600 mg), droloxifene (1-600 mg), idoxifine (1-600 mg), nafoxidine (0.5-600 mg), toremifene, TAT-59 (0.1-600 mg), levomeloxifene (0.5-600 mg), LY-353381 (1-600 mg), CP-336156, MDL-103323, EM-800, and ICI-182,780 (0.1-150 mg) with a progestin for 28 consecutive days per 28 day menstrual cycle to provide contraception.

It is preferred that the anti-estrogen plus progestin regimen be administered according to a monophasic type regimen continuously during the 28-day menstrual cycle. In a monophasic regimen, the same dose of each of the anti-estrogen and progestin are administered each day during the administration period. The continuous 28-day administration of the anti-estrogen plus progestin combination will eliminate the withdrawal bleed that is associated with other non-continuous oral contraceptive regimens, and will eliminate the irregular bleeding (breakthrough and spotting) that is associated with progestin only oral contraceptive regimens.

When the compound of Example 32 and levonorgestrel are administered according to a 28-day monophasic regimen, the following dosages are preferred, with regimen A being most preferred.

| Regimen | Example 32 | Levonorgestrel |
|---|---|---|
| A | 2 mg | 90 µg |
| B | 3 ma | 75 µg |
| C | 5 mg | 100 µg |

The anti-estrogen plus progestin contraceptives of this invention can also be administered for 28 days each menstrual cycle according to phased regimens (i.e., biphasic, triphasic, quadraphasic, and the like). In such regimen, the same dosage of the combination is administered each day of the particular phase, with each phase having a different dosage than the preceding or subsequent phase. In a typical quadraphasic regimen, each phase may be 7 days in length. The regimens may be quadraphasic rising regimens in which the dosage of antiestrogen and progestin is increased from phase I to phase II and from phase II to phase III; the dosage during the fourth phase is then typically lower than in the first phase. One skilled in the art will appreciate that this invention also covers regimens in which the dosages of the first or second phase will be highest. Other variations include maintaining a constant dosage of progestin during all four phases, while varying the dosage of antiestrogen of the four phases, with the phase III dosage typically being the highest, and phase IV dosage being the lowest. Alternatively, the dosage of antiestrogen can be held constant during all four phases, while the dosage of progestin is being varied from phase to phase.

For administration, it is preferred that the combination anti-estrogen plus progestin contraceptive be administered in unit dosage form i.e., tablet or pill, with each unit providing the entire daily dosage. It is preferred that the progestin and anti-estrogen are admixed together in the same dosage unit. Such dosage units can be prepared by conventional methodology that is well known to one skilled in the art. In each dosage unit, the contraceptively active progestin and estrogen are combined with excipients, vehicles, pharmaceutically acceptable carriers, and colorants.

This invention also provides a contraceptive kit adapted for daily oral administration which comprises a total of 28 separate dosage units. In this kit, each dosage units each consisting of a combination of progestin at a daily dosage equivalent in progestational activity to 30-150 µg levonorgestrel and an anti-estrogen at a daily dosage equivalent to 0.5-25 mg of the compound of Example 32. The daily dosage arrangements are preferably arranged in a blister pack or in a dial pack type tablet dispenser. Specific referred progestins and anti-estrogens and the specifically preferred dosages of each combination dosage unit are described above.

## Claims

1. A method of providing contraception which comprises administering to a female of childbearing age a contraceptive effective amount of a combination of an anti-estrogen of formulas I or II having the structure 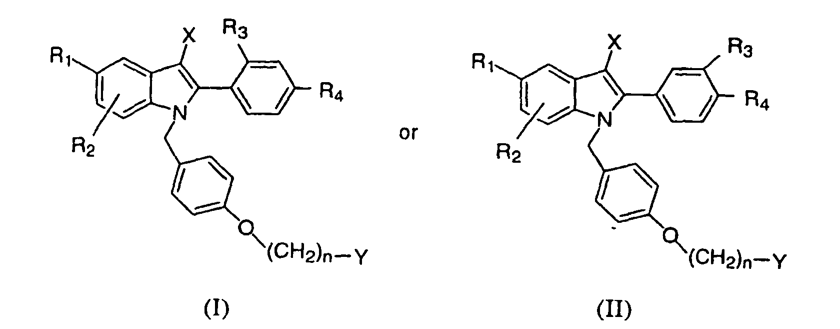 wherein:
R₁ is H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen or mono- or poly-fluoroalkoxy of 1-12 carbon atoms;
R₂ is H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen, mono- or poly-fluoroalkoxy of 1-12 carbon atoms, cyano, alkyl of 1-6 carbon atoms, or trifluoromethyl, with the proviso that, when R₁ is H, R₂ is not OH;
R₃ and R₄ are each, independently, H, OH alkoxycarbonyl or aralkoxycarbonyl of 2-12 carbon atoms, alkoxy or aralkoxy of 1-12 carbon atoms, cycloalkyloxy of 3-12 carbon atoms, halogen, mono- or poly-fluoroalkoxy of 1-12 carbon atoms, or cyano;
X is H, alkyl of 1-6 carbon atoms, cyano, nitro, trifluoromethyl, or halogen;
n is 2 or 3;
Y is a saturated, partially saturated or unsaturated 5-7 membered heterocycle containing a nitrogen, which may optionally contain a second heteroatom selected from the group consisting of -O-, -NH- , alkylamine of 1-6 carbon atoms, -N< , and S(O)ₘ;
m is 0-2;
or a pharmaceutically acceptable salt thereof,
and a progestin for 28 consecutive days per 28-day menstrual cycle.

2. The method according to claim 1 wherein
Y is

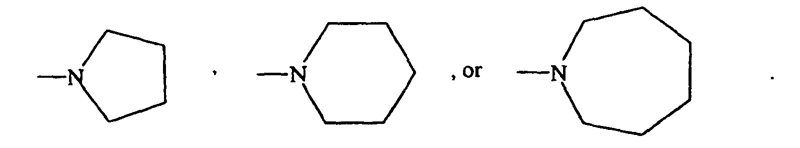

3. The method according to claim 1 wherein
R₁ is selected from H, OH, alkoxy carbonyl of 2-12 carbon atoms, alkoxy of 1-12 carbon atoms, benzyloxy, cycloalkyloxy of 3-12 carbon atoms or halogen;
R₂, R₃ and R₄ are independently selected from H, OH or alkoxycarbonyl of 2-12 carbon atoms, alkoxy of 1-12 carbon atoms, benzyloxy, cycloalkyloxy of 3-12 carbon atoms, halogen, cyano, C₁-C₆ alkyl, or trihalomethyl, preferably trifluoromethyl, with the proviso that, when R₁ is H, R₂ is not OH;
X is selected from H, C₁-C₆ alkyl, cyano, nitro, trifluoromethyl, halogen;
and Y is

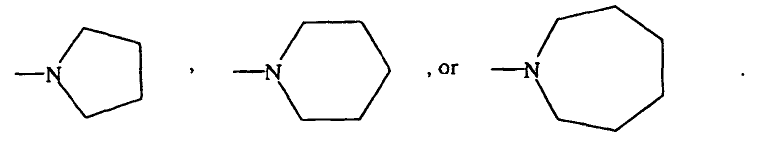

4. The method according to claim 1 wherein the progestin is selected from the group consisting of levonorgestrel, norgestrel, desogestrel, 3-ketodesogestrel, norethindrone, gestodene, norethisterone acetate, norgestimate, osaterone, cyproterone acetate, trimegestone, dienogest, and drospirenone.

5. The method according to claim 4, wherein the progestin is levonorgestrel.

6. The method according to any of the preceding claims, wherein the anti-estrogen is selected from the group consisting of
a) 5-benzyloxy-2-(4-ethoxy-phenyl)-3-methyl-1-[4-(2-piperidin-1-yl-ethoxy)-benzyl]-1H-indole or a pharmaceutically acceptable salt thereof;
b) 5-benzyloxy-2-phenyl-3-methyl-1-[4-(2-azepan-1-yl-ethoxy)-benzyl]-1H-indole or a pharmaceutically acceptable salt thereof;
c) 5-benzyloxy-2-(4-benzyloxy-phenyl)-3-methyl-1-[4-(2-piperidin-1-yl-ethoxy)-benzyl]-1H-indole or a pharmaceutically acceptable salt thereof;
d) 5-benzyloxy-2-(4-benzyloxy-phenyl)-3-methyl-1-[4-(2-azepan-1-yl-ethoxy)-benzyl]-1H-indole or a pharmaceutically acceptable salt thereof;
e) 5-benzyloxy-2-(4-fluoro-phenyl)-3-methyl-1-[4-(2-azepan-1-yl-ethoxy)-benzyl]-1H-indole or a pharmaceutically acceptable salt thereof;
f) 5-benzyloxy-2-(4-fluoro-phenyl)-3-methyl-1-[4-(2-piperidin-1-yl-ethoxy)-benzyl]-1H-indole or a pharmaceutically acceptable salt thereof;
g) 5-benzyloxy-2-(4-chloro-phenyl)-3-methyl-1-[4-(2-piperidin-1-yl-ethoxy)-benzyl]-1H-indole or a pharmaceutically acceptable salt thereof;
h) 5-benzyloxy-2-[3,4-methylenedioxy-phenyl]-3-methyl-1-[4-(2-piperidin-1-yl-ethoxy)-benzyl]-1H-indole or a pharmaceutically acceptable salt thereof;
i) 5-benzyloxy-2-[4-isopropoxy-phenyl]-3-methyl-1-[4-(2-piperidin-1-yl-ethoxy)-benzyl]-1H-indole or a pharmaceutically acceptable salt thereof;
j) 5-benzyloxy-2-[4-methyl-phenyl]-3-methyl-1-[4-(2-piperidin-1-yl-ethoxy)-benzyl]-1H-indole or a pharmaceutically acceptable salt thereof;
k) 5-benzyloxy-2-(3-benzyloxy-phenyl)-3-methyl-1-[4-(2-piperidin-1-yl-ethoxy)-benzyl]-1H-indole or a pharmaceutically acceptable salt thereof;
l) 1-[4-(2-azepan-1-yl-ethoxy)-benzyl]-5-benzyloxy-2-(3-benzyloxy-phenyl)-3-methyl-1H-indole or a pharmaceutically acceptable salt thereof;
m) 5-benzyloxy-2-(4-benzyloxy-3-fluoro-phenyl)-3-methyl-1-[4-(2-piperidin-1-yl-ethoxy)-benzyl]-1H-indole or a pharmaceutically acceptable salt thereof;
n) 5-benzyloxy-2-(4-benzyloxy-3-fluoro-phenyl)-3-methyl-1-[4-(2-azepan-1-yl-ethoxy)-benzyl]-1H-indole or a pharmaceutically acceptable salt thereof;
o) 5-benzyloxy-2-(3-methoxy-phenyl-1-[4-(2-piperidin-1-yl-ethoxy)-benzyl]-3-methyl-1H-indole or a pharmaceutically acceptable salt thereof;
p) 5-benzyloxy-3-methyl-1-[4-(2-piperidin-1-yl-ethoxy)-benzyl]-2-(4-trifluoromethoxy-phenyl)-1H-indole or a pharmaceutically acceptable salt thereof;
q) 5-benzyloxy-2-(4-benzyloxy-phenyl)-3-methyl-1-{4-methyl-piperazin-1-yl)-ethoxy]-benzyl}-1H-indole or a pharmaceutically acceptable salt thereof;
r) 1-[4-(2-azepan-1-yl-ethoxy)-benzyl]-5-benzyloxy-2-(3-methoxyphenyl)-3-methyl-1H-indole or a pharmaceutically acceptable salt thereof;
s) 4-{3-methyl-1-[4-(2-piperidin-1-yl-ethoxy)-benzyl]-1H-indole} hydrochloride;
t) 4- {3-methyl-1-[4-(2-piperidin-1-yl-ethoxy)-benzyl]-1H-indol-2-yl}-phenol hydrochloride;
u) 3-methyl-2-phenyl-1-[4-(2-piperidine-1-yl-ethoxy)-benzyl]-1H-indol-5-ol hydrochloride;
v) 4-{5-methoxy-3-methyl-1-{4-[2-(piperidin-1-yl)-ethoxy]-benzyl}-1H-indol-2-yl}-phenol or a pharmaceutically acceptable salt thereof;
w) 2-(4-methoxy-phenyl)-3-methyl-1-{4-[2-(piperidin-1-yl)-ethoxy]-benzyl}-1H-indol-5-ol or a pharmaceutically acceptable salt thereof ;
x) 5-methoxy-2-(4-methoxy-phenyl)-3-methyl-1-[4-(2-piperidin-1-yl-ethoxy)-benzyl]-1H-indole hydrochloride;
y) 1-[4-(2-azepan-1-yl-ethoxy)-benzyl]-5-methoxy-2-(4-methoxyphenyl)-3-methyl-1H-indole hydrochloride;
z) 2-(4-ethoxy-phenyl)-3-methyl-1-[4-(2-piperidin-1-yl-ethoxy)-benzyl]-1H-indol-5-ol or a pharmaceutically acceptable salt thereof;
aa) 1-[4-(2-azepan-1-yl-ethoxy)-benzyl]-2-(4-ethoxy-phenyl)-3-methyl-1H-indol-5-ol or a pharmaceutically acceptable salt thereof;
ab) 4-{5-fluoro-3-methyl-1-[4-(2-piperidin-1-yl-ethoxy)-benzyl]-1H-indol-2-yl}-phenol hydrochloride;
ac) 1-[4-(2-azepan-1-yl-ethoxy)-benzyl]-3-methyl-2-phenyl-1H-indol-5-ol hydrochloride;
ad) 2-(4-hydroxy-phenyl)-3-methyl-1-[4-(2-pyrollidin-1-yl-ethoxy)-benzyl]-1H-indol-5-ol or a pharmaceutically acceptable salt thereof;
ae) 2-(4-hydroxy-phenyl)-3-methyl-1-[4-(2-piperidin-1-yl-ethoxy)-benzyl]-1H-indol-5-ol or a pharmaceutically acceptable salt thereof;
af) 1-[4-(2-azepan-1-yl-ethoxy)-benzyl]-2-(4-hydroxy-phenyl)-3-methyl-1H-indol-5-ol hydrochloride;
ag) 2-(4-fluoro-phenyl)-3-methyl-1-[4-(2-piperidine-1-yl-ethoxy)-benzyl]-1H-indol-5-ol hydrochloride;
ah) 1-[4-(2-azepan-1-yl-ethoxy)-benzyl]-2-(4-fluoro-phenyl)-3-methyl-1H-indol-5-ol or a pharmaceutically acceptable salt thereof;
ai) 2-(3-methoxy-4-hydroxy-phenyl)-3-methyl-1-[4-(2-piperidin-1-yl-ethoxy)-benzyl]-1H-indol-5-ol hydrochloride;
aj) 2-benzo[1,3]dioxol-5-yl-3-methyl-1-[4-(2-piperidin-1-yl-ethoxy)-benzyl]-1H-indol-5-ol hydrochloride;
ak) 2-(4-isopropoxy-phenyl)-3-methyl-1-[4-(2-piperidin-1-yl-ethoxy)-benzyl]-1H-indol-5-ol hydrochloride;
al) 1-[4-(2-azepan-1-yl-ethoxy)-benzyl]-2-(4-isopropoxy-phenyl)-3-methyl-1H-indol-5-ol hydrochloride;
am) 2-(4-cyclopentyloxy-phenyl)-3-methyl-1-(4-(2-piperidin-1-yl-ethoxy)-benzyl]-1H-indol-5-ol or a pharmaceutically acceptable salt thereof;
an) 2-(4-chloro-phenyl)-3-methyl-1-[4-(2-piperidin-1-yl-ethoxy)-benzyl]-1H-indol-5-ol hydrochloride;
ao) 2-(2,4-dimethoxy-phenyl)-3-methyl-1-[4-(2-piperidin-1-yl-ethoxy)-benzyl]-1H-indol-5-ol or a pharmaceutically acceptable salt thereof;
ap) 2-(3-hydroxy-phenyl)-3-methyl-1-[4-(2-piperidin-1-yl-ethoxy)-benzyl]-1H-indol-5-ol or a pharmaceutically acceptable salt thereof;
aq) 1-[4-(2-azepan-1-yl-ethoxy)-benzyl]-2-(3-hydroxy-phenyl)-3-methyl-1H-indole-5-ol or a pharmaceutically acceptable salt thereof;
ar) 2-(3-fluoro-4-hydroxy-phenyl)-3-methyl-1-[4-(2-piperidin-1-yl-ethoxy)-benzyl]-1H-indol-5-ol or a pharmaceutically acceptable salt thereof;
as) 2-(3-fluoro-4-hydroxy-phenyl)-3-methyl-1-[4-(azepan-1-yl-ethoxy)-benzyl]-1H-indol-5-ol or a pharmaceutically acceptable salt thereof;
at) 2-(3-methoxy-phenyl)-3-methyl-1-[4-(2-piperidin-1-yl-ethoxy)-benzyl]-1H-indole-5-ol or a pharmaceutically acceptable salt thereof;
au) 3-methyl-1-[4-(2-piperidin-1-yl-ethoxy)-benzyl]-2-(4-trifluoro-methoxy-phenyl)- 1H-indole-5-ol or a pharmaceutically acceptable salt thereof;
av) 3-chloro-2-(4-hydroxy-phenyl)-1-[4-(2-pyrrolidin-1-yl-ethoxy)-benzyl]-1H-indol-5-ol hydrochloride;
aw) 3-chloro-2-(4-hydroxy-phenyl)-1-[4-(2-piperidin-1-yl-ethoxy)-benzyl]-1H-indol-5-ol hydrochloride;
ax) 3-chloro-2-(4-hydroxy-phenyl)-1-[4-(2-azepan-1-yl-ethoxy)-benzyl]- 1H-indol-5-ol hydrochloride;
ay) 3-chloro-2-(4-hydroxy-2-methyl-phenyl)-1-[4-(2-piperidin-1-yl-ethoxy)-benzyl]-1H-indol-5-ol or a pharmaceutically acceptable salt thereof;
az) 2-(4-hydroxy-phenyl)-3-ethyl-1-[4-(2-piperidin-1-yl-ethoxy)-benzyl]-1H-indol-5-ol hydrochloride;
ba) 5-hydroxy-2-(4-hydroxy-phenyl)-1-[4-(2-piperidin-1-yl-ethoxy)-benzyl]-1H-indole-3-carbonitrile hydrochloride;
bb) 1-[4-(2-azepan-1-yl-ethoxy)-benzyl]-5-hydroxy-2-(4-hydroxyphenyl)-1H-indole-3-cabonitrile hydrochloride;
bc) di-propionate of 1-[4-(2-azepan-1-yl-ethoxy)-benzyl]-2-(4-hydroxy-phenyl)-3-methyl-1H-indol-5-ol hydrochloride;
bd) di-pivalate of 1-[4-(2-azepan-1-yl-ethoxy)-benzyl]-2-(4-hydroxyphenyl)-3-methyl-1H-indol-5-ol hydrochloride;
be) di-pivalate ester of 2-(4-hydroxy-phenyl)-3-methyl-1-[4-(2-piperidin-1-yl-ethoxy)-benzyl]-1H-indol-5-ol or a pharmaceutically acceptable salt thereof;

7. The method according to any of the preceding claims, wherein the anti-estrogen is 1-[4-(2-azepan-1-yl-ethoxy)-benzyl]-2-(4-hydroxy-phenyl)-3-methyl-1H-indol-5-ol or a pharmaceutically acceptable salt thereof.

8. The method according to any of claims 1 to 5, wherein the antiestrogen is 1-[4-(2-azepan-1-yl-ethoxy)-benzyl]-2-(4-hydroxy-phenyl)-3-methyl-1H-indol-5-ol acetate.

9. The method according any of the preceding claims, wherein the same dosage of the anti-estrogen and progestin combination is administered in each of the 28 days.

10. A method of providing contraception which comprises administering to a female of child bearing age a combination of a daily dosage of 0.5 - 25 mg 1-[4-(2-azepan-1-yl-ethoxy)-benzyl]-2-(4-hydroxy-phenyl)-3-methyl-1H-indol-5-ol acetate and 30-150 µg levonorgestrel for 28 consecutive days per 28 day menstrual cycle.

11. The method according to claim 10, wherein the same dosage of the combination is administered in each of the 28 days.

12. A method of providing contraception which comprises administering to a female of child bearing age a combination of a non-uterotrophic anti-estrogen and a progestin for 28 days per 28-day menstrual cycle.

13. The method according to claim 12, wherein the progestin is selected from the group consisting of levonorgestrel, norgestrel, desogestrel, 3-ketodesogestrel, norethindrone, gestodene, norethisterone acetate, norgestimate, osaterone, cyproterone acetate, trimegestone, dienogest, and drospirenone.

14. The method according to claim 13, wherein the anti-estrogen is selected from the group consisting of raloxifene, droloxifene, idoxifine, nafoxidine, toremifene, TAT-59, levomeloxifene, LY-353381, CP-336156, MDL-103323, EM-800, and ICI-182,780.

15. The method according to claim 14, wherein the same dosage of the anti-estrogen and progestin combination is administered in each of the 28 days.

16. A contraceptive kit adapted for daily oral administration which comprises 28 separate dosage units, each containing a combination of a non-uterotrophic anti-estrogen and a progestin.

17. The kit according to claim 16, wherein the anti-estrogen is a compound of formulas I or II having the structure 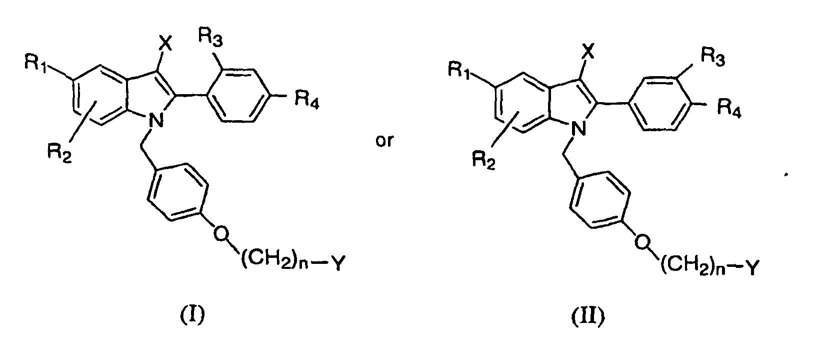 wherein:
R₁ is H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen or mono- or poly-fluoroalkoxy of 1-12 carbon atoms;
R₂ is H, OH, alkoxycarbonyl or aralkoxycarbonyl of 2-12 carbon atoms, alkoxy or aralkoxy of 1-12 carbon atoms, cycloalkyloxy of 3-12 carbon atoms, halogen, mono- or poly-fluoroalkoxy of 1-12 carbon atoms, cyano, alkyl of 1-6 carbon atoms, or trifluoromethyl, with the proviso that, when R₁ is H, R₂ is not OH;
R₃ and R₄ are each, independently, H, OH alkoxycarbonyl or aralkoxycarbonyl of 2-12 carbon atoms, alkoxy or aralkoxy of 1-12 carbon atoms, cycloalkyloxy of 3-12 carbon atoms, halogen, mono- or poly-fluoroalkoxy of 1-12 carbon atoms, or cyano;
X is H, alkyl of 1-6 carbon atoms, cyano, nitro, trifluoromethyl, or halogen; n is 2 or 3;
Y is a saturated, partially saturated or unsaturated 5-7 membered heterocycle containing a nitrogen, which may optionally contain a second heteroatom selected from the group consisting of -O-, -NH- , alkylamine of 1-6 carbon atoms, -N< , and S(O)ₘ;
m is 0-2;
or a pharmaceutically acceptable salt thereof,and the progestin is selected from the group consisting of levonorgestrel, norgestrel, desogestrel, 3-ketodesogestrel, norethindrone, gestodene, norethisterone acetate, norgestimate, osaterone, cyproterone acetate, trimegestone, dienogest, and drospirenone.

18. The kit according to claim 17, wherein the anti-estrogen is 1-[4-(2-azepan-1-yl-ethoxy)-benzyl]-2-(4-hydroxy-phenyl)-3-methyl-1H-indol-5-ol acetate and the progestin is levonorgestrel.

## Patentansprüche

1. Verfahren zur Kontrazeption, wobei man einer Frau im gebärfähigen Alter eine kontrazeptiv wirksame Menge einer Kombination eines Antiöstrogens der Formeln I oder II mit der Struktur 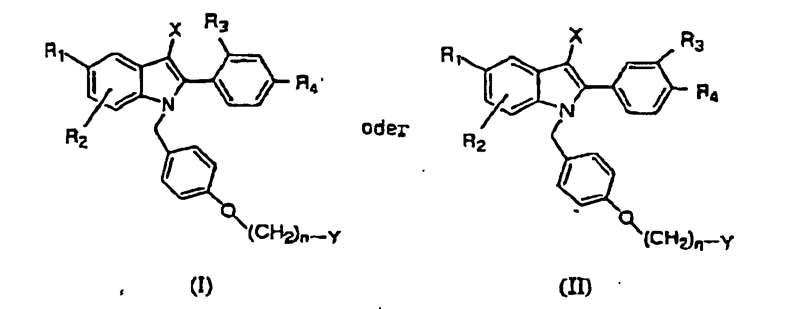 worin:
R₁ für H, OH, Alkoxycarbonyl oder Aralkoxycarbonyl mit 2 bis 12 Kohlenstoffatomen, Alkoxy oder Aralkoxy mit 1 bis 12 Kohlenstoffatomen, Cycloalkyloxy mit 3 bis 12 Kohlenstoffatomen, Halogen, oder Mono- oder Polyfluoralkoxy mit 1 bis 12 Kohlenstoffatomen steht;
R₂ für H, OH, Alkoxycarbonyl oder Aralkoxycarbonyl mit 2 bis 12 Kohlenstoffatomen, Alkoxy oder Aralkoxy mit 1 bis 12 Kohlenstoffatomen, Cycloalkyloxy mit 3 bis 12 Kohlenstoffatomen, Halogen, Mono- oder Polyfluoralkoxy mit 1 bis 12 Kohlenstoffatomen, Cyano, Alkyl mit 1 bis 6 Kohlenstoffatomen, oder Trifluormethyl steht, mit der Maßgabe, dass R₂ nicht OH ist, falls R₇ H ist;
R₃ und R₄ jeweils unabhängig voneinander für H, OH, Alkoxycarbonyl oder Aralkoxycarbonyl mit 2 bis 12 Kohlenstoffatomen, Alkoxy oder Aralkoxy mit 1 bis 12 Kohlenstoffatomen, Cycloalkyloxy mit 3 bis 12 Kohlenstoffatomen, Halogen, Mono- oder Polyfluoralkoxy mit 1 bis 12 Kohlenstoffatomen, oder Cyano stehen;
X für H, Alkyl mit 1 bis 6 Kohlenstoffatomen, Cyano, Nitro, Trifluormethyl, oder Halogen steht;
n 2 oder 3 ist;
Y für einen gesättigten, partiell gesättigten oder ungesättigten, 5-7-gliedrigen, ein Stickstoffatom enthaltenden Heterocyclus steht, der gegebenenfalls ein unter -O-, -NH-, Alkylamin mit 1 bis 6 Kohlenstoffatomen, -N< und S(O)ₘ ausgewähltes zweites Heteroatom enthalten kann;
m 0-2 ist;
oder ein pharmazeutisch akzeptables Salz davon,
und ein Progestin an 28 aufeinanderfolgenden Tagen eines 28-tägigen Menstruationszyklus verabreicht.

2. Verfahren nach Anspruch 1, wobei
Y 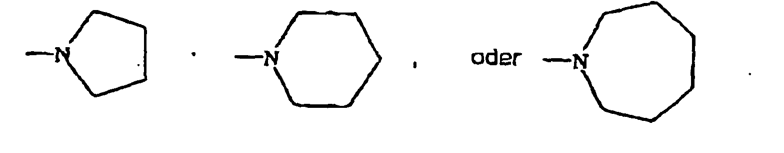 ist.

3. Verfahren nach Anspruch 1, wobei
R₁ ausgewählt ist unter H, OH, Alkoxycarbonyl mit 2 bis 12 Kohlenstoffatomen, Alkoxy mit 1 bis 12 Kohlenstoffatomen, Benzyloxy, Cycloalkyloxy mit 3 bis 12 Kohlenstoffatomen, oder Halogen;
R₂, R₃ und R₄ unabhängig voneinander ausgewählt sind unter H, OH oder Alkoxycarbonyl mit 2 bis 12 Kohlenstoffatomen, Alkoxy mit 1 bis 12 Kohlenstoffatomen, Benzyloxy, Cycloalkyloxy mit 3 bis 12 Kohlenstoffatomen, Halogen, Cyano, C₁-C₆-Alkyl, oder Trihalomethyl, vorzugsweise Trifluormethyl, mit der Maßgabe, dass R₂ nicht OH ist, falls R₁ H ist;
X ausgewählt ist unter H, C₁-C₆-Alkyl, Cyano. Nitro, Trifluormethyl, Halogen;
und
Y 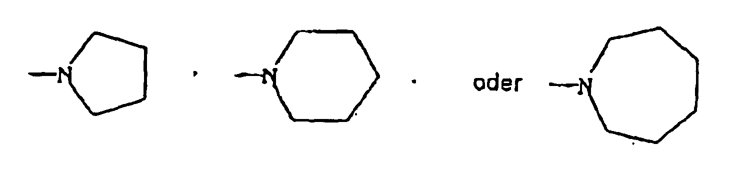 ist.

4. Verfahren nach Anspruch 1, wobei das Progestin ausgewählt ist unter Levonorgestrel, Norgestrel, Dasogestrel, 3-Ketodesogestrel, Norethindron, Gestoden, Norethisteronacetat, Norgestimat, Osateron, Cyproteronacetat, Trimegeston, Dienogest und Drospirenon.

5. Verfahren nach Anspruch 4, wobei das Progestin Levonorgestrel ist.

6. Verfahren nach einem der vorhergehenden Ansprüche, wobei das Antiöstrogen ausgewählt ist unter
a) 5-Benzyloxy-2-(4-ethoxy-phenyl)-3-methyl-1-[4-(2-piperidin-1-yl-ethoxy)-benzyl]-1H-indol oder ein pharmazeutisch akzeptables Salz davon;
b) 5-Benzyloxy-2-phenyl-3-methyl-1-[4-(2-azepan-1-yl-ethoxy)-benzyl]-1H-indol oder ein pharmazeutisch akzeptables Salz davon;
c) 5-Benzyloxy-2-(4-benzyloxy-phenyl)-3-methyl-1-[4-(2-piperidin-1-yl-ethoxy)-benzyl]-1H-indol oder ein pharmazeutisch akzeptables Salz davon;
d) 5-Benzyloxy-2-(4-benzyloxy-phenyl)-3-methyl-1-[4-(2-azepen-1-yl-ethoxy)-benzyl]-1H-indol oder ein pharmazeutisch akzeptables Salz davon;
e) 5-Benzyloxy-2-(4-fluor-phenyl)-3-methyl-1-[4-(2-azepan-1-yl-ethoxy)-benzyl]-1H-indol oder ein pharmazeutisch akzeptables Salz davon;
f) 5-Benzyloxy-2-(4-fluor-phenyl)-3-methyl-1-[4-(2-piperidin-1-yl-ethoxy)-benzyl]-1H-indol oder ein pharmazeutisch akzeptables Salz davon;
g) 5-Benzyloxy-2-(4-chlor-phenyl)-3-methyl-1-[4-(2-piperidin-1-yl-ethoxy)-benzyl]-1H-indol oder ein pharmazeutisch akzeptables Salz davon;
h) 5-Benzyloxy-2-[3,4-methylendioxy-phenyl]-3-methyl-1-[4-(2-piperidin-1-yl-ethoxy)-benzyl]-1H-indol oder ein pharmazeutisch akzeptables Salz davon;
i) 5-Benzyloxy-2-[4-isopropoxy-phenyl]-3-methyl-1-[4-(2-piperidin-1-yl-ethoxy)-benzyl]-1H-indol oder ein pharmazeutisch akzeptables Salz davon;
j) 5-Benzyloxy-2-[4-methyl-phenyl]-3-methyl-1-[4-(2-piperidin-1-yl-ethoxy)-benzyl]-1H-indol oder ein pharmazeutisch akzeptables Salz davon;
k) 5-Benzyloxy-2-(3-benzyloxy-phenyl)-3-methyl-1-[4-(2-piperidin-1-yl-ethoxy)-benzyl]-1H-indol oder ein pharmazeutisch akzeptables Salz davon;
l) 1-[4-(2-Azepan-1-yl-ethoxy)-benzyl]-5-benzyloxy-2-(3-benzyloxyphenyl)-3-methyl-1H-indol oder ein pharmazeutisch akzeptables Salz davon;
m) 5-Benzyloxy-2-(4-benzyloxy-3-fluor-phenyl)-3-methyl-1-[4-(2-piperidin-1-yl-ethoxy)-benzyl]-1H-indol oder ein pharmazeutisch akzeptables Salz davon;
n) 5-Benzyloxy-2-(4-benzyloxy-3-fluor-phenyl)-3-methyl-1-[4-(2-azepan-1-yl-ethoxy)-benzyl]-1H-indol oder ein pharmazeutisch akzeptables Salz davon;
o) 5-Benzyloxy-2-(3-methoxy-phenyl-1-[4-(2-piperidin-1-yl-ethoxy)-benzyl]-3-methyl-1 H-indol oder ein pharmazeutisch akzeptables Salz davon;
p) 5-Benzyloxy-3-methyl-1-[4-(2-piperidin-1-yl-ethoxy)-benzyl]-2-(4-trifluormethoxy-phenyl)-1H-indol oder ein pharmazeutisch akzeptables Salz davon;
q) 5-Benzyloxy-2-(4-benzyloxy-phenyl)-3-methyl-1-{4-methyl-piperazin-1-yl)-ethoxy]-benzyl}-1H-indol oder ein pharmazeutisch akzeptables Salz davon;
r) 1-[4-(2-Azepan-1-yl-ethoxy)-benzyl]-5-benzyloxy-2-(3-methoxy-phenyl)-3-methyl-1H-indol oder ein pharmazeutisch akzeptables Salz davon;
s) 4-{3-Methyl-1-[4-(2-piperidin-1-yl-ethoxy)-benzyl]-1H-indol)-hydrochlorid;
t) 4-{3-Methyl-1-[4-(2-piperidin-1-yl-ethoxy)-benzyl]-1H-indol-2-yl}-phenol-hydrochlorid;
u) 3-Methyl-2-phenyl-1-[4-(2-piperidin-1-yl-ethoxy)-benzyl]-1H-indol-5-ol-hydrochlorid;
v) 4-{5-Methoxy-3-methyl-1-{4-[2-(piperidin-1-yl)-ethoxy]-benzyl}-1H-indol-2-yl}-phenol oder ein pharmazeutisch akzeptables Salz davon;
w) 2-(4-Methoxy-phenyl)-3-methyl-1-{4-[2-(piperidin-1-yl)-ethoxy]-benzyl}-1 H-indol-5-ol oder ein pharmazeutisch akzeptables Salz davon;
x) 5-Methoxy-2-(4-methoxy-phenyl)-3-methyl-1-[4-(2-piperidin-1-yl-ethoxy)-benzyl]-1H-indol-hydrochlorid;
y) 1-[4-(2-Azepan-1-yl-ethoxy)-benzyl]-5-methoxy-2-(4-methoxy-phenyl)-3-methyl-1-indol-hydrochlorid;
z) 2-(4-Ethoxy-phenyl)-3-methyl-1-[4-(2-piperidin-1-yl-ethoxy)-benzyl]-1H-indol-5-ol oder ein pharmazeutisch akzeptables Salz davon;
aa) 1-[4-(2-Azepan-1-yl-ethoxy)-benzyl]-2-(4-ethoxy-phenyl)-3-methyl-1H-indol-5-ol oder ein pharmazeutisch akzeptables Salz davon;
ab) 4-{5-Fluor-3-methyl-1-[4-(2-piperidin-1-yl-ethoxy)-benzyl]-1H-indol-2-yl}-phenol-hydrochlorid;
ac) 1-[4-(2-Azepan-1-yl-ethoxy)-benzyl]-3-methyl-2-phenyl-1H-indol-5-ol-hydrochlorid;
ad) 2-(4-Hydroxy-phenyl)-3-methyl-1-[4-(2-pyrrolidin-1-yl-ethoxy)-benzyl]-1H-indol-5-ol oder ein pharmazeutisch akzeptables Salz davon;
ae) 2-(4-Hydroxy-phenyl)-3-methyl-1-[4-(2-piperidin-1-yl)-ethoxy)-benzyl]-1 H-indol-5-ol oder ein pharmazeutisch akzeptables Salz davon;
af) 1-[4-(2-Azepan-1-yl-ethoxy)-benzyl]-2-(4-hydroxy-phenyl)-3-methyl-1H-indol-5-ol-hydrochlorid;
ag) 2-(4-Fluor-phenyl)-3-methyl-1-[4-(2-piperidin-1-yl-ethoxy)-benzyl]-1H-indol-5-ol-hydrochlorid;
ah) 1-[4-(2-Azepan-1-yl-ethoxy)-benzyl]-2-(4-fluor-phenyl)-3-methyl-1H-indol-5-ol oder ein pharmazeutisch akzeptables Salz davon;
ai) 2-(3-Methoxy-4-hydroxy-phenyl)-3-methyl-1-[4-(2-piperidin-1-yl-ethoxy)-benzyl]-1H-indol-5-ol-hydrochlorid;
aj) 2-Benzo[1,3]dioxol-5-yl-3-methyl-1-[4-(2-piperidin-1-yl-ethoxy)-benzyl]-1 H-indol-5-ol-hydrochlorid;
ak) 2-(4-Isopropoxy-phenyl)-3-methyl-1-[4-(2-piperidin-1-yl-ethoxy)-benzyl]-1 H-indol-5-ol-hydrochlorid;
al) 1-[4-(2-Azepan-1-yl-ethoxy)-benzyl]-2-(4-isopropoxy-phenyl)-3-methyl-1 H-indol-5-ol-hydrochloild;
am) 2-(4-Cyclopentyloxy-phenyl)-3-methyl-1-[4-(2-piperidin-1-yl-ethoxy)-benzyl]-1H-indol-5-ol oder ein pharmazeutisch akzeptables Salz davon;
an) 2-(4-Chlor-phenyl)-3-methyl-1-[4-(2-piperidin-1-yl-ethoxy)-benzyl]-1H-indol-5-ol-hydrochlorid;
ao) 2-(2,4-Dimethoxy-phenyl)-3-methyl-1-[4-(2-piperidin-1-yl-ethoxy)-benzyl]-1H-indol-5-ol oder ein pharmazeutisch akzeptables Salz davon;
ap) 2-(3-Hydroxy-phenyl)-3-methyl-1-[4-(2-piperidin-1-yl-ethoxy)-benzyl]-1 H-indol-5-ol oder ein pharmazeutisch akzeptables Salz davon;
aq) 1-[4-(2-Azepan-1-yl-ethoxy)-benzyl]-2-(3-hydroxy-phenyl)-3-methyl-1H-indol-5-ol oder ein pharmazeutisch akzeptables Salz davon;
ar) 2-(3-Fluor-4-hydroxy-phenyl)-3-methyl-1-[4-(2-piperidin-1-yl-ethoxy)-benzyl]-1H-indol-5-ol oder ein pharmazeutisch akzeptables Salz davon:
as) 2-(3-Fluor-4-hydroxy-phenyl)-3-methyl-1-(4-(azepan-1-yl-ethoxy)-benzyl]-1H-indol-5-ol oder ein pharmazeutisch akzeptables Salz davon;
at) 2-(3-Methoxy-phenyl)-3-methyl-1-[4-(2-piperidin-1-yl-ethoxy)-benzyl]-1H-indol-5-ol oder ein pharmazeutisch akzeptables Salz davon:
au) 3-Methyl-1-[4-(2-piperidin-1-yl-ethoxy)-benzyl]-2-(4-trifluor-methoxyphenyl)-1H-indol-5-ol oder ein pharmazeutisch akzeptables Salz davon;
av) 3-Chlor-2-(4-hydroxy-phenyl)-1-[4-(2-pyrrolidin-1-yl-ethoxy)-benzyl]-1H-indol-5-ol-hydrochlorid;
aw) 3-Chlor-2-(4-hydroxy-phenyl)-1-[4-(2-piperidin-1-yl-ethoxy)-benzyl]-1H-indol-5-ol-hydrochlorid;
ax) 3-Chlor-2-(4-hydroxy-phenyl)-1-[4-(2-azepan-1-yl-ethoxy)-benzyl]-1H-indol-5-ol-hydrochlorid;
ay) 3-Chlor-2-(4-hydroxy-2-methyl-phenyl)-1-[4-(2-piperidin-1-yl-ethoxy)-benzyl]-1H-indol-5-ol oder ein pharmazeutisch akzeptables Salz davon;
az) 2-(4-Hydroxy-phenyl)-3-ethyl-1-[4-(2-piperidin-1-yl-ethoxy)-benzyl]-1H-indol-5-ol-hydrochlorid;
ba) 5-Hydroxy-2-(4-hydroxy-phenyl)-1-[4-(2-piperidin-1-yl-ethoxy)-benzyl]-1 H-indol-3-carbonitril-hydrochlorid;
bb) 1-[4-(2-Azepan-1-yl-ethoxy)-benzyl]-5-hydroxy-2-(4-hydroxy-phenyl)-1 H-indol-3-carbonitril-hydrochlorid;
bc) Dipropionat von 1-[4-(2-Azepan-1-yl-ethoxy)-benzyl]-2-(4-hydroxyphenyl)-3-methyl-1 H-indol-5-ol-hydrochlorid;
bd) Dipivalat von 1-[4-(2-Azepan-1-yl-ethoxy)-benzyl]-2-(4-hydroxy-phenyl)-3-methyl-1H-indol-5-ol-hydrochlorid;
be) Dipivalatester von 2-(4-Hydroxy-phenyl)-3-methyl-1-[4-(2-piperidin-1-yl-ethoxy)-benzyl]-1H-indol-5-ol oder ein pharmazeutisch akzeptables Salz davon;

7. Verfahren nach einem der vorhergehenden Ansprüche, wobei das Antiöstrogen 1-[4-(2-Azepan-1-yl-ethoxy)-benzyl]-2-(4-hydroxy-phenyl)-3-methyl-1 H-indol-5-ol oder ein pharmazeutisch akzeptables Salz davon ist.

8. Verfahren nach einem der Ansprüche 1 bis 5, worin das Antiöstrogen 1-[4-(2-Azepan-1-yl-ethoxy)-benzyl]-2-(4-hydroxy-phenyl)-3-methyl-1H-indol-5-ol-acetat ist.

9. Verfahren nach einem der vorhergehenden Ansprüche, wobei an jedem der 28 Tage die gleiche Dosierung der Antiöstrogen- und Progestin-Kombination verabreicht wird.

10. Verfahren zur Kontrazeption, wobei man einer Frau im gebärfähigen Alter eine Kombination einer täglichen Dosierung von 0.5-25 mg 1-[4-(2-Azepan-1-yl-ethoxy)-benzyl]-2-(4-hydroxy-phenyl)-3-methyl-1H-indol-5-ol-acetat und 30-150 µg Levonorgestrel an 28 aufeinanderfolgenden Tagen eines 28-tägigen Menstruationszyklus verabreicht.

11. Verfahren nach Anspruch 10, wobei an jedem der 28 Tage die gleiche Dosierung der Kombination verabreicht wird.

12. Verfahren zur Kontrazeption, wobei man einer Frau im gebärfähigen Alter eine Kombination eines nicht-uterotrophen Antiöstrogens und eines Progestins an 28 Tagen eines 28-tägigen Menstruationszyklus verabreicht.

13. Verfahren nach Anspruch 12, wobei das Progestin ausgewählt ist unter Levonorgestrel, Norgestrel, Desogestrel, 3-Kelodesogestrel, Norethindron, Gestoden, Norethisteronacetat, Norgestimat, Osateron, Cyproteronacetat, Trimegeston, Dienogest und Drospirenon.

14. Verfahren nach Anspruch 13, wobei das Antiöstrogen ausgewählt ist unter Raloxifen, Droloxifen, Idoxifin, Nafoxidin, Toremifen, TAT-59, Levomeloxifen, LY-353381, CP-336156, MDL-103323, EM-800, und ICI-182,780.

15. Verfahren nach Anspruch 14, wobei an jedem der 28 Tage die gleiche Dosierung der Antiöstrogen- und Progestin-Kombination verabreicht wird.

16. Kontrazeptionskit, der für die tägliche orale Verabreichung ausgelegt ist, umfassend 28 getrennte Dasierungseinheiten, die jeweils eine Kombination eines nicht-uterotrophen Antiöstrogens und eines Progestins enthalten.

17. Kit nach Anspruch 16, wobei das Antiöstrogen eine Verbindung der Formeln I oder II mit der Struktur 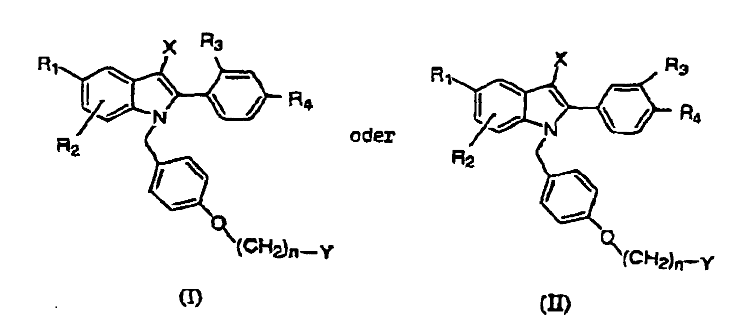 ist, worin:
R₁ für H, OH, Alkoxycarbonyl oder Aralkoxycarbonyl mit 2 bis 12 Kohlenstoffatomen, Alkoxy oder Aralkoxy mit 1 bis 12 Kohlenstoffatomen, Cycloalkyloxy mit 3 bis 12 Kohlenstoffatomen, Halogen, oder Mono- oder Polyfluoralkoxy mit 1 bis 12 Kohlenstoffatomen steht;
R₂ für H, OH, Alkoxycarbonyl oder Aralkoxycarbonyl mit 2 bis 12 Kohlenenffatomen, Alkoxy oder Aralkoxy mit 1 bis 12 Kohlenstoffatomen, Cycloalkyloxy mit 3 bis 12 Kohlenstoffatomen, Halogen, Mono- oder Polyfluoralkoxy mit 1 bis 12 Kohlenstoffatomen, Cyano, Alkyl mit 1 bis 6 Kohlenstoffatomen, oder Trifluormethyl steht, mit der Maßgabe, dass R₂ nicht OH ist, falls R₁ H ist;
R₃ und R₄ jeweils unabhängig voneinander für H, OH, Alkoxycarbonyl oder Aralkoxycarbonyl mit 2 bis 12 Kohlenstoffatomen, Alkoxy oder Aralkoxy mit 1 bis 12 Kohlenstoffatomen, Gycloalkyloxy mit 3 bis 12 Kohlenstoffatomen, Halogen, Mono- oder Polyfluoralkoxy mit 1 bis 12 Kohlenstoffatomen, oder Cyano stehen;
X für H, Alkyl mit 1 bis 6 Kohlenstoffatomen, Cyano, Nitro, Trifluormethyl oder Halogen steht;
n 2 oder 3 ist
Y für einen gesättigten, partiell gesättigten oder ungesättigten, 5-7-gliedrigen, ein Stickstoffatom enthaltenden Heterocyclus steht, der gegebenenfalls ein unter -O-, -NH-, Alkylamin mit 1 bis 6 Kohlenstoffatomen, -N< und S(O)ₘ ausgewähltes zweites Heteroatom enthalten kann;
m 0-2 ist;
oder ein pharmazeutisch akzeptables Salz davon,
und das Progestin ausgewählt ist unter Levonorgestrel, Norgestrel, Desogestrel, 3-Ketodesogestrel, Norethindron, Gestoden, Norethisteronacetat, Norgestimat, Osateron, Cyproteronacetat, Trimegeston, Dienogest und Drospirenon.

18. Kit nach Anspruch 17, wobei das Antiöstrogen 1-[4-(2-Azepan-1-yl-ethoxy)-benzyl]-2-(4-hydroxy-phenyl)-3-methyl-1H-indol-5-ol-acetat ist und das Progestin Levonorgestrel ist.

## Revendications

1. Procédé de contraception, qui comprend l'administration à un sujet féminin en âge de procréer d'une quantité, efficace du point de vue contraceptif, d'une association d'un anti-oestrogène de formule I ou II ayant la structure 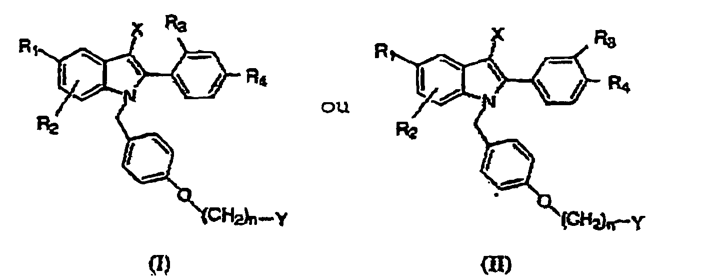 dans laquelle :
R₁ représente H, un groupe OH, alkoxycarbonyle ou aralkoxycarbonyle de 2 à 12 atomes de carbone, alkoxy ou aralkoxy de 1 à 12 atomes de carbone, cycloalkyloxy de 3 à 12 atomes de carbone, halogéno ou mono- ou poly-fluoralkoxy de 1 à 12 atomes de carbone ;
R₂ représente H, un groupe OH, alkoxycarbonyle ou aralkoxycarbonyle de 2 à 12 atomes de carbone, alkoxy ou aralkoxy de 1 à 12 atomes de carbone, cycloalkyloxy de 3 à 12 atomes de carbone, halogéno, mono- ou poly-fluoralkoxy de 1 à 12 atomes de carbone, cyano, alkyle de 1 à 6 atomes de carbone, ou trifluorométhyle, sous réserve que, lorsque R₁ représente H, R₂ ne représente pas un groupe OH ;
R₃ et R₄ représentent chacun, indépendamment, H, un groupe OH; alkoxycarbonyle ou aralkoxycarbonyle de 2 à 12 atomes de carbone, alkoxy ou aralkoxy de 1 à 12 atomes de carbone, cycloalkyloxy de 3 à 12 atomes de carbone, halogéno, mono- ou polyfluoralkoxy de 1 à 12 atomes de carbone ou cyano ;
X représente H, un groupe alkyle de 1 à 6 atomes de carbone, cyano, nitro, trifluorométhyle, ou halogéno ;
n est égal à 2 ou 3 ;
Y représente un hétérocycle penta- à heptagonal saturé, partiellement saturé ou insaturé contenant un atome d'azote, qui peut contenir facultativement un second hétéroatome choisi dans le groupe consistant en -O-, -NH-, alkylamine de 1 à 6 atomes de carbone, -N<, et S(O)ₘ ;
m a une valeur de 0 à 2 ;
ou un de ses sels pharmaceutiquement acceptables ;
et d'une hormone progestative pendant 28 jours consécutifs par cycle menstruel de 28 jours.

2. Méthode suivant la revendication 1, dans laquelle
Y représente un groupe

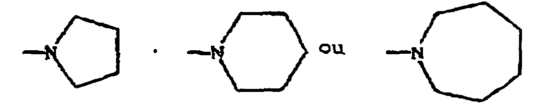

3. Méthode suivant la revendication 1, dans laquelle R₁ est choisi entre H, des groupes OH, alkoxycarbonyle de 2 à 12 atomes de carbone, alkoxy de 1 à 12 atomes de carbone, benzyloxy, cycloalkyloxy de 3 à 12 atomes de carbone ou halogéno ;
R₂, R₃ et R₄ sont choisis indépendamment entre H, des groupes OH, alkoxycarbonyle de 2 à 12 atomes de carbone, alkoxy de 1 à 12 atomes de carbone, benzyloxy, cycloalkyloxy de 3 à 12 atomes de carbone, halogéno, cyano, alkyle en C₁ à C₆ ou trihalogénométhyle, de préférence trifluorométhyle, sous réserve que, lorsque R₁ représente H, R₂ ne représente pas un groupe OH ;
X est choisi entre H, des groupes alkyle en C₁ à C₆, cyano, nitro, trifluorométhyle et halogéno;
et Y représente un groupe

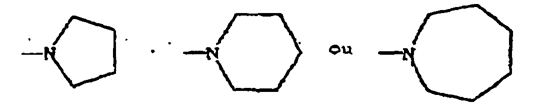

4. Méthode suivant la revendication 1, dans laquelle l'hormone progestative est choisie dans le groupe consistant en lévonorgestrel, norgestrel, désogestrel, 3-cétodésogestrel, noréthindrone, gestodène, acétate de noréthistérone, norgestimate, osatérone, acétate de cyprotérone, trimégestone, diénogest, et drospirénone.

5. Méthode suivant la revendication 4, dans laquelle l'hormone progestative est le lévonorgestrel.

6. Méthode suivant l'une quelconque des revendications précédentes, dans laquelle l'anti-oestrogène est choisi dans le groupe consistant en :
a) le 5-benzyloxy-2-(4-éthoxy-phényl)-3-méthyl-1-[4-(2-pipéridine-1-yl-éthoxy)-benzyl]-1H-indole ou un de ses sels pharmaceutiquement acceptables ;
b) le 5-benzyloxy-2-phényl-3-méthyl-1-[4-(2-azépan-1-yl-éthoxy)-benzyl]-1H-indole ou un de ses sels pharmaceutiquement acceptables ;
c) le 5-benzyloxy-2-(4-benzyloxy-phényl)-3-méthyl-1-[4-(2-pipéridine-1-yl-éthoxy)-benzyl]-1H-indole ou un de ses sels pharmaceutiquement acceptables ;
d) le 5-benzyloxy-2-(4-benzyloxy-phényl)-3-méthyl-1-[4-(2-azépan-1-yl-éthoxy)-benzyl]-1H-indole ou un de ses sels pharmaceutiquement acceptables ;
e) le 5-benzyloxy-2-(4-fluoro-phényl)-3-méthyl-1-[4-(2-azépan-1-yl-éthoxy)-benzyl]-1H-indole ou un de ses sels pharmaceutiquement acceptables ;
f) le 5-benzyloxy-2-(4-fluoro-phényl)-3-méthyl-1-[4-(2-pipéridine-1-yl-éthoxy)-benzyl]-1H-indole ou un de ses sels pharmaceutiquement acceptables ;
g) le 5-benzyloxy-2-(4-chloro-phényl)-3-méthyl-1-[4-(2-pipéridine-1-yl-éthoxy)-benzyl]-1H-indole ou un de ses sels pharmaceutiquement acceptables ;
h) le 5-benzyloxy-2-[3,4-méthylènedioxy-phényl]-3-méthyl-1-[4-(2-pipéridine-1-yl-éthoxy)-benzyl]-1H-indole ou un de ses sels pharmaceutiquement acceptables ;
i) le 5-benzyloxy-2-[4-isopropoxy-phényl)-3-méthyl-1-[4-(2-pipéridine-1-yl-éthoxy)-benzyl]-1H-indole ou un de ses sels pharmaceutiquement acceptables ;
j) le 5-benzyloxy-2-[4-méthyl-phényl]-3-méthyl-1-[4-(2-pipéridine-1-yl-éthoxy)-benzyl]-1H-indole ou un de ses sels pharmaceutiquement acceptables ;
k) le 5-benzyloxy-2-(3-benzyloxy-phényl)-3-méthyl-1-[4-(2-pipéridine-1-yl-éthoxy)-benzyl]-1H-indole ou un de ses sels pharmaceutiquement acceptables ;
l) le 1-[4-(2-azépan-1-yl-éthoxy)-benzyl]-5-benzyloxy-2-(3-benzyloxy-phényl)-3-méthyl-1H-indole ou un de ses sels pharmaceutiquement acceptables ;
m) le 5-benzyloxy-2-(4-benzyloxy-3-fluoro-phényl)-3-méthyl-1-[4-(2-pipéridine-1-yl-éthoxy)-benzyl]-1H-indole ou un de ses sels pharmaceutiquement acceptables ;
n) le 5-benzyloxy-2-(4-benzyloxy-3-fluoro-phényl)-3-méthyl-1-[4-(2-azépan-1-yl-éthoxy)-benzyl]-1H-indole ou un de ses sels pharmaceutiquement acceptables ;
o) le 5-benzyloxy-2-(3-méthoxy-phényl-1-[4-(2-pipéridine-1-yl-éthoxy)-benzyl]-3-méthyl-1H-indole ou un de ses sels pharmaceutiquement acceptables ;
p) le 5-benzyloxy-3-méthyl-1-[4-(2-pipéridine-1-yl-éthoxy)-benzyl]-2-(4-trifluorométhoxy-phényl)-1H-indole ou un de ses sels pharmaceutiquement acceptables ;
q) le 5-benzyloxy-2-(4-benzyloxy-phényl)-3-méthyl-1-{4-méthyl-pipérazine-1-yl)-éthoxy]-benzyl}-1H-indole ou un de ses sels pharmaceutiquement acceptables ;
r) le 1-[4-(2-azépan-1-yl-éthoxy)-benzyl]-5-benzyloxy-2-(3-méthoxy-phényl)-3-méthyl-1H-indole ou un de ses sels pharmaceutiquement acceptables ;
s) le chlorhydrate de 4-{3-méthyl-1-[4-(2-pipéridine-1-yl-éthoxy)-benzyl]-1H-indole} ;
t) le chlorhydrate de 4-{3-méthyl-1-[4-(2-pipéridine-1-yl-éthoxy)-benzyl]-1H-indole-2-yl}-phénol ;
u) le chlorhydrate de 3-méthyl-2-phényl-1-[4-(2-pipéridine-1-yl-éthoxy)-benzyl]-1H-indole-5-ol ;
v) le 4-{5-méthoxy-3-méthyl-1-{4-[2-(pipéridine-1-yl)-éthoxy]-benzyl}-1H-indole-2-yl}-phénol ou un de ses sels pharmaceutiquement acceptables ;
w) le 2-(4-méthoxy-phényl)-3-méthyl-1-{4-[2-(pipéridine-1-yl)-éthoxy]-benzyl}-1H-indole-5-ol ou un de ses sels pharmaceutiquement acceptables ;
x) le chlorhydrate de 5-méthoxy-2-(4-méthoxy-phényl)-3-méthyl-1-[4-(2-pipéridine-1-yl-éthoxy)-benzyl]-1H-indole ;
y) le chlorhydrate de 1-[4-(2-azépan-1-yl-éthoxy)-benzyl]-5-méthoxy-2-(4-méthoxy-phényl)-3-méthyl-1H-indole ;
z) le 2-(4-éthoxy-phényl)-3-méthyl-1-[4-(2-pipéridine-1-yl-éthoxy)-benzyl]-1H-indole-5-ol ou un de ses sels pharmaceutiquement acceptables ;
aa) le 1-[4-(2-azépan-1-yl-éthoxy)-benzyl]-2-(4-éthoxy-phényl)-3-méthyl-1H-indole-5-ol ou un de ses sels pharmaceutiquement acceptables ;
ab) le chlorhydrate de 4-{5-fluoro-3-méthyl-1-[4-(2-pipéridine-1-yl-éthoxy)-benzyl]-1H-indole-2-yl}-phénol ;
ac) le chlorhydrate de 1-[4-(2-azépan-1-yl-éthoxy)-benzyl]-3-méthyl-2-phényl-1H-indole-5-ol ;
ad) le 2-(4-hydroxy-phényl)-3-méthyl-1-[4-(2-pyrollidine-1-yl-éthoxy)-benzyl]-1H-indole-5-ol ou un de ses sels pharmaceutiquement acceptables ;
ae) le 2-(4-hydroxy-phényl)-3-méthyl-1-[4-(2-pipéridine-1-yl-éthoxy)-benzyl]-1H-indole-5-ol ou un de ses sels pharmaceutiquement acceptables ;
af) le chlorhydrate de 1-[4-(2-azépan-1-yl-éthoxy)-benzyl]-2-(4-hydroxy-phényl)-3-méthyl-1H-indole-5-ol ;
ag) le chlorhydrate de 2-(4-fluoro-phényl)-3-méthyl-1-[4-(2-pipéridine-1-yl-éthoxy)-benzyl]-1H-indole-5-ol ;
ah) le 1-[4-(2-azépan-1-yl-éthoxy)-benzyl]-2-(4-fluoro-phényl)-3-méthyl-1H-indole-5-ol ou un de ses sels pharmaceutiquement acceptables ;
ai) le chlorhydrate de 2-(3-méthoxy-4-hydroxy-phényl)-3-méthyl-1-[4-(2-pipéridine-1-yl-éthoxy)-benzyl]-1H-indole-5-ol ;
aj) le chlorhydrate de 2-benzo[1,3]dioxol-5-yl-3-méthyl-1-[4-(2-pipéridine-1-yl-éthoxy)-benzyl]-1H-indole-5-ol ;
ak) le chlorhydrate de 2-(4-isopropoxy-phényl)-3-méthyl-1-[4-(2-pipéridine-1-yl-éthoxy)-benzyl]-1H-indole-5-ol ;
al) le chlorhydrate de 1-[4-(2-azépan-1-yl-éthoxy)-benzyl]-2-(4-isopropoxy-phényl)-3-méthyl-1H-indole-5-ol ;
am) le 2-(4-cyclopentyloxy-phényl)-3-méthyl-1-(4-(2-pipéridine-1-yl-éthoxy)-benzyl]-1H-indole-5-ol ou un de ses sels pharmaceutiquement acceptables ;
an) le chlorhydrate de 2-(4-chloro-phényl)-3-méthyl-1-[4-(2-pipéridine-1-yl-éthoxy)-benzyl]-1H-indole-5-ol ;
ao) le 2-(2,4-diméthoxy-phényl)-3-méthyl-1-[4-(2-pipéridine-1-yl-éthoxy)-benzyl]-1H-indole-5-ol ou un de ses sels pharmaceutiquement acceptables ;
ap) le 2-(3-hydroxy-phényl)-3-méthyl-1-(4-(2-pipéridine-1-yl-éthoxy)-benzyl]-1H-indole-5-ol ou un de ses sels pharmaceutiquement acceptables ;
aq) le 1-[4-(2-azépan-1-yl-éthoxy)-benzyl]-2-(3-hydroxy-phényl)-3-méthyl-1H-indole-5-ol ou un de ses sels pharmaceutiquement acceptables ;
ar) le 2-(3-fluoro-4-hydroxy-phényl)-3-méthyl-1-[4-(2-pipéridine-1-yl-éthoxy)-benzyl]-1H-indole-5-ol ou un de ses sels pharmaceutiquement acceptables ;
as) le 2-(3-fluoro-4-hydroxy-phényl)-3-méthyl-1-[4-(azépan-1-yl-éthoxy)-benzyl]-1H-indole-5 ol ou un de ses sels pharmaceutiquement acceptables ;
at) le 2-(3-méthoxy-phényl)-3-méthyl-1-[4-(2-pipéridine-1-yl-éthoxy)-benzyl]-1H-indole-5-ol ou un de ses sels pharmaceutiquement acceptables ;
au) le 3-méthyl-1-[4-(2-pipéridine-1-yl-éthoxy)-benzyl]-2-(4-trifluorométhoxy-phényl)-1H-indole-5-ol ou un de ses sels pharmaceutiquement acceptables ;
av) le chlorhydrate de 3-chloro-2-(4-hydroxy-phényl)-1-[4-(2-pyrrolidine-1-yl-éthoxy)-benzyl]-1H-indole-5-ol ;
aw) le chlorhydrate de 3-chloro-2-(4-hydroxy-phényl)-1-[4-(2-pipéridine-1-yl-éthoxy)-benzyl]-1H-indole-5-ol ;
ax) le chlorhydrate de 3-chloro-2-(4-hydroxy-phényl)-1-[4-(2-azépan-1-yl-éthoxy)-benzyl]-1H-indole-5-ol ;
ay) le 3-chloro-2-(4-hydroxy-2-méthyl-phényl)-1-[4-(2-pipéridine-1-yl-éthoxy)-benzyl]-1H-indole-5-ol ou un de ses sels pharmaceutiquement acceptables ;
az) le chlorhydrate de 2-(4-hydroxy-phényl)-3-éthyl-1-[4-(2-pipéridine-1-yl-éthoxy)-benzyl]-1H-indole-5-ol ;
ba) le chlorhydrate de 5-hydroxy-2-(4-hydroxy-phényl)-1-[4-(2-pipéridine-1-yl-éthoxy)-benzyl]-1H-indole-3-carbonitrile ;
bb) le chlorhydrate de 1-[4-(2-azépan-1-yl-éthoxy)-benzyl]-5-hydroxy-2-(4-hydroxy-phényl)-1H-indole-3-carbonitrile ;
bc) le dipropionate de chlorhydrate de 1-[4-(2-azépan-1-yl-éthoxy)-benzyl]-2-(4-hydroxy-phényl)-3-méthyl-1H-indole-5-ol ;
bd) le dipivalate de chlorhydrate de 1-[4-(2-azépan-1-yl-éthoxy)-benzyl]-2-(4-hydroxy-phényl)-3-méthyl-1H-indole-5-ol ;
be) l'ester dipivalate de 2-(4-hydroxy-phényl)-3-méthyl-1-[4-(2-pipéridine-1-yl-éthoxy)-benzyl]-1H-indole-5-ol ou un de ses sels pharmaceutiquement acceptables.

7. Méthode suivant l'une quelconque des revendications précédentes, dans laquelle l'anti-oestrogène est le 1-[4-(2-azépan-1-yl-éthoxy)-benzyl]-2-(4-hydroxy-phényl)-3-méthyl-1H-indole-5-ol ou un de ses sels pharmaceutiquement acceptables.

8. Méthode suivant l'une quelconque des revendications 1 à 5, dans laquelle l'anti-oestrogène est l'acétate de 1-[4-(2-azépan-1-yl-éthoxy)-benzyl]-2-(4-hydroxy-phényl)-3-méthyl-1H-indole-5-ol.

9. Méthode suivant l'une quelconque des revendications précédentes, dans laquelle la même dose de l'association d'anti-oestrogènes et d'hormones progestatives est administrée à chacun des 28 jours.

10. Méthode de contraception, qui comprend l'administration à un sujet féminin en âge de procréer d'une association d'une dose quotidienne de 0,5 à 25 mg d'acétate de 1-[4-(2-azépan-1-yl-éthoxy)-benzyl]-2-(4-hydroxy-phényl)-3-méthyl-1H-imdole-5-ol et de 30 à 150 µg de lévonorgestrel pendant 28 jours consécutifs par cycle menstruel de 28 jours.

11. Méthode suivant la revendication 10, dans laquelle la même dose de l'association est administrée à chacun des 28 jours.

12. Méthode de contraception, qui comprend l'administration à un sujet féminin en âge de procréer d'une association d'un anti-oestrogène non utérotrophe et d'une hormone progestative pendant 28 jours par cycle menstruel de 28 jours.

13. Méthode suivant la revendication 12, dans laquelle l'hormone progestative est choisie dans le groupe consistant en lévonorgestrel, norgestrel, désogestrel, 3-cétodésogestrel, noréthindrone, gestodène, acétate de noréthistérone, norgestimate, osatérone, acétate de cyprotérone, trimégestone, diénogest et drospirénone.

14. Méthode suivant la revendication 13, dans laquelle l'anti-oestrogène est choisi dans le groupe consistant en raloxifène, droloxifène, idoxifine, nafoxidine, torémifène, TAT-59, lévoméloxifène, LY-353381, CP-336156, MDL-103323, EM-800, et ICI-182,780.

15. Méthode suivant la revendication 14, dans laquelle la même dose de l'association d'anti-oestrogènes et d'hormones progestatives est administrée à chacun des 28 jours.

16. Kit contraceptif adapté à l'administration orale quotidienne, qui comprend 28 doses unitaires distinctes, chacune contenant une association d'un anti-oestrogène non utérotrophe et d'une hormone progestative.

17. Kit suivant la revendication 16, dans lequel l'anti-oestrogène est un composé de formule I ou II ayant la structure 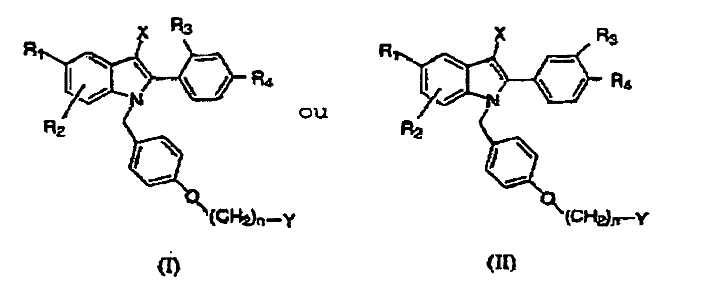 dans laquelle :
R₁ représente H, un groupe OH, alkoxycarbonyle ou aralkoxycarbonyle de 2 à 12 atomes de carbone, alkoxy ou aralkoxy de 1 à 12 atomes de carbone, cycloalkyloxy de 3 à 12 atomes de carbone, halogéno ou mono- ou polyfluoralkoxy de 1 à 12 atomes de carbone ;
R₂ représente H, un groupe OH, alkoxycarbonyle ou aralkoxycarbonyle de 2 à 12 atomes de carbone, alkoxy ou aralkoxy de 1 à 12 atomes de carbone, cycloalkyloxy de 3 à 12 atomes de carbone, halogéno, mono- ou polyfluoralkoxy de 1 à 12 atomes de carbone, cyano, alkyle de 1 à 6 atomes de carbone, ou trifluorométhyle, sous réserve que, lorsque R₁ représente H, R₂ ne représente pas un groupe OH ;
R₃ et R₄ représentent chacun, indépendamment, H, un groupe OH, alkoxycarbonyle ou aralkoxycarbonyle de 2 à 12 atomes de carbone, alkoxy ou aralkoxy de 1 à 12 atomes de carbone, cycloalkyloxy de 3 à 12 atomes de carbone, halogéno, mono- ou polyfluoralkoxy de 1 à 12 atomes de carbone ou cyano ;
X représente H, un groupe alkyle de 1 à 6 atomes de carbone, cyano, nitro, trifluorométhyle, ou halogéno ;
n est égal à 2 ou 3 ;
Y représente un hétérocycle penta- à heptagonal saturé, partiellement saturé ou insaturé contenant un atome d'azote, qui peut contenir facultativement un second hétéroatome choisi dans le groupe consistant en -O-, -NH-, alkylamine de 1 à 6 atomes de carbone, -N<, et S(O)ₘ ;
m a une valeur de 0 à 2 ;
ou un de ses sels pharmaceutiquement acceptables, et l'hormone progestative est choisie dans le groupe consistant en lévonorgestrel, norgestrel, désogestrel, 3-cétodésogestrel, noréthindrone, gestodène, acétate de noréthistérone, norgestimate, osatérone, acétate de cyprotérone, trimégestone, diénogest et drospirénone.

18. Kit suivant la revendication 17, dans lequel l'anti-oestrogène est l'acétate de 1-[4-(2-azépan-1-yl-éthoxy)-benzyl]-2-(4-hydroxy-phényl)-3-méthyl-1H-indole-5-ol et l'hormone progestative est le lévonorgestrel.
